# EUROPEAN PATENT APPLICATION

(11) **EP 4 338 740 A1**
(43) Date of publication of application: **20.03.2024**
(21) Application number: 22195291.4
(22) Date of filing: 13.09.2022
(51) Int. Cl.: A61K 31/522, A61K 9/70

(54) **A TRANSDERMAL PATCH FOR PROMOTING OR ACCELERATING MYELINATION AND/OR REMYELINATION**

(71) Applicant: Johannes Gutenberg-Universität Mainz, 55122 Mainz (DE)
(72) Inventor: JACOB, Claire, 55116 Mainz (DE); LANGGUTH, Peter, 55099 Mainz (DE); AL-GOUSOUS, Jozef, 55118 Mainz (DE); ENGEL, Sophia, 65185 Wiesbaden (DE)
(74) Representative: Patentanwälte Dr. Keller, Schwertfeger Partnerschaft mbB

(57) **Abstract**

The present invention relates to a transdermal patch comprising a backing layer, a matrix layer comprising aminophylline or theophylline or a salt thereof, a matrix polymer, a plasticizer, a permeation enhancer, and a removable protective release liner for use in the treatment or prevention of a hypomyelinating or a demyelinating disease or condition or a lesion of the peripheral or central nervous system where demyelination occurs. The invention further relates to a pharmaceutical composition comprising aminophylline or theophylline, a matrix polymer, a plasticizer and a permeation enhancer for use as a medicament for promoting or accelerating myelination and/or remyelination use in the treatment or prevention of a hypomyelinating or a demyelinating disease or condition or a lesion of the peripheral or central nervous system where demyelination occurs.

## Description

### Field of the Invention

The present invention relates to a transdermal patch comprising aminophylline or theophylline, a matrix polymer, a plasticizer and a permeation enhancer for use in the treatment or prevention of a demyelinating disease or demyelinating lesion. The invention further relates to a pharmaceutical composition comprising said compounds for use as a medicament for promoting or accelerating myelination and/or re-myelination.

### Background of the Invention

Demyelinating diseases belong to diseases of the nervous system in which the myelin sheath of neurons is damaged. This damage impairs the conduction of signals in the affected nerves. In turn, the reduction in conduction causes deficiency in sensation, movement, cognition, or other functions depending on which nerves are affected. The demyelinating disease usually includes diseases affecting the central nervous system and peripheral nervous system. Examples of such demyelinating diseases of the peripheral nervous system include Guillain-Barre syndrome and Charcot Marie Tooth (CMT) disease, whereas demyelinating diseases of the central nervous system include multiple sclerosis.

Demyelinating diseases may be caused by overexpression of peripheral myelin protein 22 (PMP22). PMP22 is a protein which in humans is encoded by the PMP22 gene. The integral membrane protein encoded by this gene is a hydrophobic, tetraspan glycoprotein expressed mainly in Schwann cells and is a major component of compact myelin in the peripheral nervous system. Various mutations of the gene are causes of CMT1A, Dejerine-Scottas disease, and hereditary neuropathy with liability to pressure palsy (HNPP).

Demyelinating lesions include, but are not limited to lesions of the nervous system myelin sheath, traumatic lesions of the nervous system, peripheral nerve injury or spinal cord injury. Myelin sheath is a layer of lipid cell membrane that covers sheath nerve fiber axon outside and consists of myelin sheath cells, which main physiological function is to act as an insulation and protection functions on nerve axon, and facilitates rapid transmission of nervous impulse.

It has been shown that a high concentration of theophylline is required to reach the dermis in order to be effective (Touitou et al., International Journal of Pharmaceutics (1991), Vol: 70, 1-2).

The effect of oleic acid, propylene glycol and transcutol on the skin permeation fluxes of theophylline from various carrier systems (PEG base, cream, gel and ointment) was measured, indicating that PEG base containing oleic acid and transcutol is the best enhancing carrier for theophylline (yielding a 260-times flux increase), among the systems investigated. This enhancing formulation was found to deliver to the dermis radiolabeled theophylline when applied to rat skin in vivo and visualized by autoradiography.

Therapeutic approaches are known to treat demyelinating diseases or conditions. Furthermore, transdermal formulations are known that contain theophylline and salbutamol sulfate (SS) using hydroxypropylmethylcellulose (HPMC) (Murthy et al., Drug Dev Ind Pharm (2001) 10:1057-62).

US 2013/116205 A1 describes a composition comprising an iridoid compound for the treatment or prophylaxis of a demyelinating disease of the nervous system, in particular a disease associated with nervous system myelin sheath lesion. US 10,413,559 B2 uses carba-cyclic phosphatidic acids or salts thereof to treat a demyelinating disease. Another approach is described in EP 1 317 285 A1 which relates to the use of an effective amount of a neutralizing agent specific for interferon inducible protein of 10 kDa (IP-10).

Other approaches use transdermal patches for the treatment of demyelinating diseases such as described in US 6,306,845 B1, WO 8702583 A1, US 2007/276021 A1 or US 2008/089861 A1.

WO2006044206A2 discloses a transdermal drug delivery system characterized by the presence of a pressure sensitive adhesive and an occlusive backing layer having a high moisture vapor transmission rate. It discloses aminophylline and theophylline in paragraph [00244]. Furthermore, "enhancers" are mentioned in paragraph [00393] on pages 72-73.

US 10576043B2 discloses a method of making a sweat-resistant drug delivery system, which involves a baking step at 80° and involves further process parameters. The drug delivery system is supposed to be suitable for use with different active compounds. It provides delivery during 8-24 hours up to a maximum of 120 hours. The patch is occlusive or substantially impermeable to theophylline and other xanthine compounds mentioned. Most likely, these compounds are trapped in the polymer matrix and therefore not delivered.

WO2018224650A1 discloses the use of theophylline for promoting remyelination, but not in the context of a transdermal patch.

US 2004138239A1 discloses a transdermal formulation for topical administration for directing a pharmacologically active substance such as theophylline to a cerebral circulation for treating headache.

US 20030152612 discloses a multilayer, flexible patch for the transdermal delivery of an active ingredient that includes theophylline or aminophylline for reducing human cellulite.

The known transdermal patches and formulations suffer from the need to be administered on a daily basis, and they do not allow a constant delivery of steady drug amounts. Usually, the drug provokes an initial peak and then the amount of the drug is progressively decreased, which is detrimental for treating a demyelinating disease. Furthermore, those compositions do not promote or accelerate myelination or remyelination of lesions in the peripheral nervous system (PNS) or central nervous system (CNS).

### Description of the Invention

Against this background, it is the object of the present invention to provide a transdermal patch which is able to promote and/or accelerate myelination and/or remyelination for use in the treatment or prevention of a hypomyelinating or demyelinating disease or condition or a lesion where demyelination occurs, such as a traumatic lesion of the nervous system.

This object is solved by the subject-matter of claim 1, in particular by a transdermal patch comprising a backing layer, a matrix layer comprising aminophylline or theophylline or a salt thereof, a matrix polymer, a plasticizer, a permeation enhancer, and a removable protective for use in the treatment or prevention of a hypomyelinating or a demyelinating disease or condition or a lesion of the peripheral or central nervous system where demyelination occurs.

The transdermal patch of the present invention allows a constant slow delivery of pharmaceutically or therapeutically effective amounts of aminophylline or theophylline, or a pharmaceutically active salt thereof. Suitable aminophylline or theophylline compounds include, but are not limited to any naturally isolated or artificially manufactured compound of aminophylline or theophylline, or a pharmaceutically active salt thereof. In a first embodiment, the transdermal patch comprises aminophylline, which is physiologically converted into theophylline *in vivo.* Theophylline can be measured in the blood plasma in blood samples taken from a human or animal subject. A plasmatic concentration between 0.3 and 2.5 µg/ml is preferred in order to promote myelination and/or remyelination. In an alternative embodiment, the transdermal patch of the invention comprises theophylline, which is delivered continuously over a prolonged period of time, preferably at least 2 days, more preferably over a period of 4 consecutive days.

One advantage of the present invention is that the plasmatic concentration of theophylline varies between 0.3 and 2.5 µg/ml, a low dose which has been proven to be effective for myelination and remyelination. Most importantly, a single patch is sufficient for drug delivery over a period of 4 consecutive days or longer. The use of low concentrations of theophylline in the blood plasma is preferred because higher concentrations may not be as efficient on myelination and/or remyelination, and may result in severe secondary side effects. A single transdermal patch therefore allows a full treatment round to promote and/or accelerate myelination and/or remyelination in the treatment of a hypomyelinating or demyelinating disease or condition or a traumatic lesion. The invention makes it possible that only one transdermal patch may be used every week or every two weeks, which significantly improves the compliance of a patient, i.e., a human or animal subject, as compared to a patient that receives a daily treatment.

The transdermal patch is preferably configured to release aminophylline or theophylline or a salt thereof over a period of up to 4 consecutive days, or longer, through the skin of a human or animal subject. Preferably the patch is configured to release aminophylline or theophylline or a salt thereof over a period of at least 2 consecutive days, preferably at least 4 consecutive days. In a preferred embodiment, the transdermal patch contains aminophylline as active ingredient and delivers at least 27 mg aminophylline or 22 mg theophylline per day to an adult human subject for at least 2 consecutive days. This quantity needs to be adapted for children and animal subjects.

In a further aspect of the present invention, the transdermal patch is configured to deliver a myelination-specific or remyelination-specific dose of aminophylline to provide a plasmatic concentration between 0.3 µg/ml and 2.5 µg/ml of theophylline in blood plasma of a human or animal patient. Preferably, the aminophylline is present in said transdermal patch in an amount ranging from about 10% to about 60% by dry weight in the matrix layer, more preferably ranging from about 25% to 35% by dry weight in the matrix layer. The invention also includes interim values of any ranges mentioned in the context of the present invention.

According to the present invention, the transdermal patch comprises a multi-layer structure, which is composed of a backing layer, a matrix layer and a removable protective release liner,

The backing layer of this multi-layer structure protects the transdermal patch from outside against contamination. In preferred embodiments, the backing layer of the transdermal patch is a film, a sheet, a sheet-like porous product, a sheet-like foaming product, a fabric, a woven fabric, or a non-woven fabric made of a synthetic resin selected from the group consisting of polyethylene, polypropylene, polybutadiene, ethylene-vinyl acetate copolymers, vinyl acetatevinyl chloride copolymers, polyvinyl chloride, polyvinyl acetate, polyvinylidene chloride, polyethylene, polyethylene terephthalate, polyimide, polyester, nylon, cellulose derivatives, and polyurethane, as well as a laminate of the foregoing. In alternative embodiments, the backing layer may also be a paper or an aluminium sheet, or may consist of composite materials thereof.

The drug-containing matrix layer of the transdermal patch of the invention comprises aminophylline or theophylline, or a salt thereof, i.e. an aminophylline or theophylline salt. In addition, the matrix layer comprises a matrix polymer, a plasticizer and a permeation enhancer.

Preferably, said matrix polymer is selected from the group consisting of a cellulose polymer, hydroxypropyl methylcellulose (HPMC), hydroxypropyl methylcellulose acetate succinate (HPMC-AS), ethyl cellulose (EC), hydroxypropyl cellulose (HPC), microcrystalline cellulose (MCC). Any suitable hydroxypropyl cellulose, hypromellose, methycellulose, ethylcellulose or sodiumcarboxymethylcellulose can be used. In some embodiments, a vinyl or acrylic polymer can be used as matrix polymer. HPMC or HPC may be available at different viscosity grades, with a molecular weight (MW) grade ranging from 20 to 1.500 kDa. Examples of such HPMC are HPMC 603, HPMC 645, HPMC 605, HPMC 606 or HPMC 615.

Preferably, HPMC is present as a matrix polymer in the transdermal patch of the present invention in an amount ranging from about 30 to 65% by dry weight in the matrix layer, preferably 35 to 60 % by dry weight in the matrix layer, more preferably about 40 to 55% by dry weight in the matrix layer. An amount of 45 to 50% by dry weight in the matrix layer is most preferred in the transdermal patch of the present invention. In an alternative embodiment, HPMC with other polymers can be used, such as polyvinylpyrrolidone (PVP).

The matrix layer of the transdermal patch of the present invention further comprises a plasticizer, which is preferably selected from the group consisting of ethylene glycol, propylene glycol, 1,2-butylene glycol, 2,3-butylene glycol, styrene glycol, polyethylene glycol, glycol ether, propylene glycol monoethyl ether, ethylene glycol monoethyl ether, diethylene glycol monoethyl ether, sorbitol lactate, ethyl lactate, butyl lactate, ethyl glycolate, allyl glycolate, monoethanolamine, diethanolamine, triethanolamine, monisopropanolamine, triethylenetetramine, 2-amino-2-methyl-1,3-propanediol, triethyl citrate, triacetyl glycerol. Plasticizers are low molecular weight resins or liquids, which cause a reduction in polymerpolymer chain secondary bonding, forming secondary bonds with the polymer chains instead. Plasticizers improve film forming properties and the appearance of the film, decreasing the glass transition temperature of the polymer, thereby preventing film cracking, increasing film flexibility and obtaining desirable mechanical properties. In addition, the plasticizer of the present invention allows a controlled release rate of aminophylline or theophylline over several days. Preferably, the transdermal patch of the present invention comprises phthalate esters, phosphate esters, fatty acid esters and glycol derivatives. Preferred plasticizers of the present invention include, but are not limited to PEG300, PEG400, PEG600, PEG800, PEG3350. Preferably, the plasticizers of the present invention are used in an amount ranging from 5% to 25% by dry weight in the matrix layer, preferably ranging from 8% to 20% by dry weight in the matrix layer, more preferably ranging from 10% to 18% by dry weight in the matrix layer.

The matrix layer of the transdermal patch of the present invention also comprises a permeation enhancer to increase permeation of aminophylline or theophylline through the skin. Permeation enhancers interact with the skin to ultimately increase drug passage through the skin. In particular, these compounds allow delivery of hydrophilic and lipophilic drugs across the skin.

It has been surprisingly found by the inventors of the present invention that a constant slow delivery of aminophylline or theophylline is achieved by using a monoterpene as permeation enhancer. Monoterpenes belong to the class of terpenes and consist of two isoprene units. In the context of the present invention, acyclic, bicyclic or monocyclic monoterpenes can be used. In addition, also modified terpenes can be used in the context of the present invention. Preferably, the monoterpene is a fatty monoterpene, such as for example aliphatic monoterpenes, in particular myrcene, ocimene. In another embodiment, cyclic monoterpenes can be used, such as, for example, alpha-pinene, camphene, limonene, phellandrene, pinene, sabinene, terpinene. In an alternative embodiment, aromatic monoterpenes can be used, including, but not limited to cymene. In a further alternative embodiment, alcoholic monoterpenes can be used, such as, for example, alcoholic monoterpenes, in particular borneol, carveol, dihydrocarveol, geraniol, menthol, linalool, perilla alcohol, sabinene hydrate, terpineol. In another embodiment, monoterpenes with carbonyl group can be used, including, but not limited to camphor, menthone, carvone, citral, cuminaldehyde, dihydrocarvone, fenchone, safranal, thujone. In an alternative embodiment, phenolic monoterpenes can be used, such as, for example, anethole, carvacrol, eugenol, eucalyptol, methyl cavicol, thymol, trans-anethole, picrocrocin. In yet another preferred embodiment, monoterpenes-related compounds or modified terpenes can be used, including, but not limited to methylbutyryloxy-1-propenylbenzene, allyltetramethoxybenzene, anisaldehyde, anisketone, apiol, elemicine, hydroxyanetholmethylbutyric acid ester, zingerone, phenylpropanes, 5-methoxyl-(2-methylbutyryloxy)-1-propenylbenzene, allyltetramethoxybenzene, anethole, cuminaldehyde, elemicin, estragole, eugenol, eucalyptol, foeniculin, hydroxyanetholmethylbutyric acid ester, methylchavicol, myristicin, safrole, trans- Anethole, Zingeron.

The use of alcoholic monoterpenes is preferred in the context of the present invention. One preferred monoterpene to be used in the present invention is geraniol. In other embodiments, a solvent, a lactam, a fatty acid, a fatty acid derivative, an amino acid derivative, α-tocopherol, and/or d-α-tocopheryl polyethylene glycol 1000 succinate can be used as a permeation enhancer. In a preferred embodiment, the solvent is glycerol, propylene glycol, dimethylsulfoxide, N-methylpyrrolidone, 2-(2-ethoxyethoxy)ethanol, dimethylformamide. Preferably, said lactam is azone or N-dodecylpyrrolidone.

In addition, other components may be added to the transdermal patch formulation of the present intervention such as, but not limited to, preservatives and antioxidants. Said preservatives may be selected from, for example, ethyl p-hydroxy benzoate, propyl p-hydroxybenzoate, butyl p-hydroxy benzoate. Said antioxidants may be selected from, for example, tocopherol and its ester derivates, ascorbic acid, ascorbic acid-stearic acid ester, dibutyl hydroxy toluene (BHT), butyl hydroxy anisole (BHA) or sodium metasulfite.

The transdermal patch of the present invention also comprises a removable protective release liner, which protects the transdermal patch during storage and is removed before application to the skin. The removable protective release liner may preferably comprise one or more of polyester, polyvinyl chloride, polypropylene, polyamide, polyvinylchloride, aluminium or polyethylenglycol terephthalate film. In preferred embodiments, the removable protective release liner is impermeable to the drug but is also easy to detach. Furthermore, the protective layer may be treated with one or more of silicone, fluorosilicone, fluorocarbon and polyethylene if required.

In a preferred embodiment, the transdermal patch of the invention comprises an additional skin adhesive layer which adheres the transdermal patch components to the skin. The adhesive layer is usually laminated on one surface of the backing layer, and a peelable film is laminated on the other surface of the adhesive layer as necessary. The backing layer supports the adhesive layer.

A preferred transdermal patch of the present invention comprises aminophylline or, alternatively, theophylline and HPMC as matrix polymer, and/or PEG as plasticizer and/or geraniol as permeation enhancer. If the transdermal patch of the present invention is composed of aminophylline, HPMC, PEG and a monoterpene such as geraniol, HPMC is present in an amount ranging from 30% to 65% by dry weight in the matrix layer, said PEG is present in an amount ranging from 5% to 25% by dry weight in the matrix layer, and said monoterpene or geraniol is present in an amount ranging from 3% to 20% by dry weight in the matrix layer.

The transdermal patch can be used for any demyelinating disease or demyelinating lesion, because it promotes or accelerates myelination or remyelination of affected nerves of a patient.

Demyelinating diseases result in a loss of myelin, which can cause neurological defects, such as vision changes, weakness, altered sensation and behavioral or cognitive problems. The symptoms of demyelination correspond to the affected area of the nervous system. Demyelination affecting the lower spine or the spinal nerves causes sensory changes or weakness of the legs. It may also be associated with a bowel and bladder control. Demyelination in the brain can cause a variety of problems, such as impaired memory or decreased vision.

The transdermal patch can be used for the treatment or prevention of any hypomyelinating or demyelinating disease or condition. Preferably, said hypomyelinating or demyelinating disease or condition is selected from the group consisting of leukodystrophy, demyelinating diseases of the central nervous system, multiple sclerosis, central pontine myelinolysis, glioma, schizophrenia, demyelination due to aging, diabetes or due to toxic agents, chronic inflammatory demyelinating polyneuropathy (CIDP), Charcot- Marie-Tooth disease (CMT), Guillain-Barre syndrome, hereditary neuropathy with liability to pressure palsy (HNPP), progressive inflammatory neuropathy, Dejerine-Sottas disease, Waardenburg syndrome, congenital hypomyelinating neuropathy (CHN), Cowchock syndrome, Rosenberg-Chutorian syndrome, or Roussy- Levy syndrome, demyelination after traumatic lesion of the PNS or CNS.

The use of the transdermal patch for the treatment of a demyelinating disease affecting the peripheral nervous system, including, but not limited to Guillain-Barre syndrome or Charcot-Marie-Tooth (CMT) disease, or the treatment of a demyelinating disease affecting the central nervous system, including, but not limited to multiple sclerosis, is preferred.

The transdermal patch of the present invention can also be used in the treatment of a demyelinating lesion, including, but not limited to traumatic lesion of the nervous system, peripheral nerve injury or spinal cord injury.

The invention also relates to a pharmaceutical composition, comprising aminophylline or theophylline, a matrix polymer, a plasticizer and a permeation enhancer for use as a medicament for promoting or accelerating myelination and/or remyelination. The matrix polymer, plasticizer or permeation enhancer of the pharmaceutical composition may be composed as described in the context of the transdermal patch of the present invention.

The invention is illustrated in more detail in the following example. By no means shall the invention be limited to this example.

### Example:

### Preparation of transdermal patch

21.875 g of HPMC 606 16% solution was weighed in an amber glass container with stirring stick. 1.05 g of PEG 300 was added to the HPMC solution and mixed until a homogeneous solution was obtained. 2.15 g aminophylline was added and allowed to mix for approx. 2 hours. 700 mg Geraniol was added and let mix for another 2h for a homogenous distribution. The suspension was poured into a petri dish and placed in a drying oven at 32°C until the water has evaporated. The patch was removed from the drying oven when it could be easily detached from the petri dish without leaving residue in the bottom. The patch was detached and stored in the closed petri dish until use.

An embodiment of the transdermal patch of the present invention has the following composition:

| **Substance** | **Quantity** | **Proportion** | **Proportion in final product after drying** | **Function** |
|---|---|---|---|---|
| Aminophylline | 2.15 g | 8.34% | 29.05% | Active ingredient |
| HPMC 606 (added as a 16 % aqueous solution) | 21.875 g | 84.87% | 47.30% | Matrix substance/ Polymere |
| PEG 300 | 1.05 g | 4.07% | 14.19% | Plasticizer |
| Geraniol | 0.7 g | 2.72% | 9.46% | Permeation enhancer |

### In vitro skin permeation test method

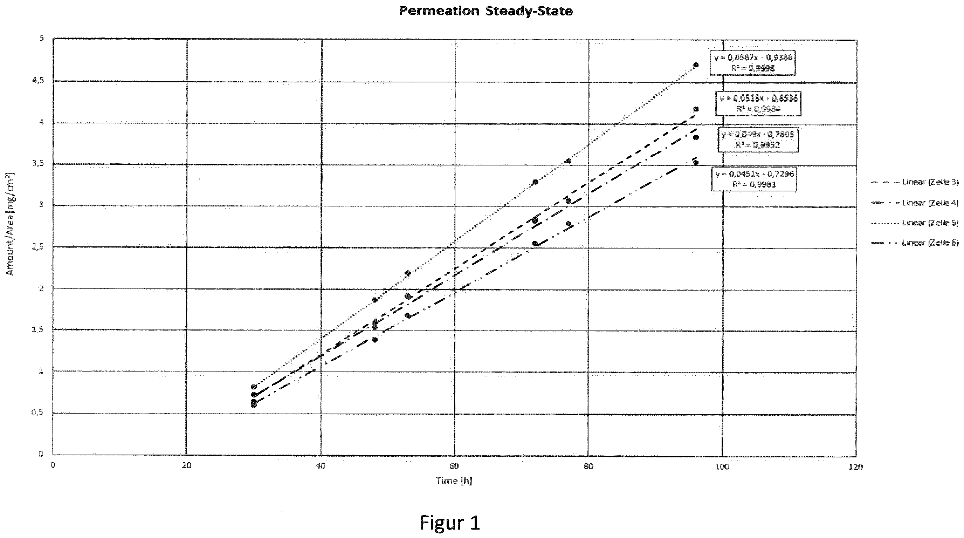

The skin permeation data given in Fig. 1 was obtained using the following test method with a diffusion cell apparatus (Model: EDC-07, Electrolab India PVT. LTD.).

The lower part of the vertical diffusion cells was filled with PBS pH 7,4 as acceptor medium. Across the orifice of the lower part of the diffusion cell pig cadaver skin was placed so that the acceptor medium contacted the skin. The medium was then tempered to 32±2°C.

A 2.01 cm² patch was punched out of the prepared patch and placed on top of the pig cadaver skin after temperature regulation. The sampling port was covered except when in use. The cells were maintained at 32±2°C throughout the course of the experiment. The acceptor fluid was stirred by means of a magnetic stirrer throughout the experiment to assure a uniform sample and reduced diffusion barrier on the dermal side of the skin. Samples were taken after 3, 6, 24, 30, 48, 53, 72, 77 and 96 hours and replaced with fresh acceptor medium.

The samples were analysed for theophylline concertation using high performance liquid chromatography with an aminophylline calibration curve and theobromine as internal standard (Column: 250x4.0 mm, 7 µm particle size, Mobile phase: 7:97 acetonitrile: 1.36 g/L solution of sodium acetate containing 0.50 per cent V/V of glacial acetic acid, Flow rate: 2.0 ml/min, Detector: UV at 272 nm, Injection volume: 20 µL, Run time: 10 minutes) The cumulative amount of theophylline penetrating through the skin was calculated and reported as µg/cm²

From this data steady state plasma concentration was calculated assuming a patch area of 60 cm² and an average theophylline clearance of 3 L/h. Fig.1 shows data of cells 3 to 6. The data demonstrate that aminophylline is constantly delivered over the skin, resulting in a steady plasma concentration of > 0.90 µg/ml, and an average flux of 51.15 µg/cm² × hour.

The data further demonstrate that the formulation is suitable for myelination and/or remyelination due to a constant release of the optimal dose in the plasma. By contrast to known transdermal patches, the formulation of the present invention needs not to be administered every day, but steady plasmatic concentration of theophylline can be maintained for at least 4 consecutive days or longer. Known transdermal patches need to be applied every day, whereas the inventive transdermal patch needs to be applied every week or every two weeks only. As such, the transdermal patch of the present invention is suitable for any treatment of a demyelinating disease or lesion involving the promotion or acceleration of myelination and/or remyelination, such as after a traumatic lesion of the nervous system, peripheral nerve injury or a spinal cord injury.

## Claims

1. A transdermal patch comprising:
a. a backing layer
b. a matrix layer comprising aminophylline or theophylline or a salt thereof, a matrix polymer, a plasticizer, a permeation enhancer
c. a removable protective release liner
for use in the treatment or prevention of a hypomyelinating or a demyelinating disease or condition or a lesion of the peripheral or central nervous system where demyelination occurs.

2. The transdermal patch for the use according to claim 1, wherein said patch comprises an additional skin adhesive layer.

3. The transdermal patch for the use according to claim 1 and 2, wherein said patch is configured to release aminophylline, theophylline or a salt thereof over a period of up to 4 consecutive days, or longer, through the skin of a human or animal subject.

4. The transdermal patch for the use according to any one of claims 1 to 3, wherein said patch is configured to deliver a myelination-specific or remyelination-specific dose of aminophylline to provide a plasmatic concentration between 0.3 µg/ml and 2.5 µg/ml of theophylline in blood plasma of a human or animal subject.

5. The transdermal patch for the use according to any one of claims 1 to 4, wherein said matrix polymer is selected from the group consisting of a cellulose polymer (hydroxypropyl methylcellulose (HPMC), hydroxypropyl methylcellulose acetate succinate (HPMC-AS), ethyl cellulose (EC), hydroxypropyl cellulose (HPC), microcrystalline cellulose (MCC)), a polymer or copolymer of acrylic acid, methacrylic acid, an acrylic acid ester and/or methacrylic acid ester residues, a polymer or copolymer of vinylpyyrolidone, vinyl alcohol and/or vinyl acetate, polyisobutylenes, polysiloxanes, polyurethanes, and silicon polymers.

6. The transdermal patch for the use according to any one of claims 1 to 5, wherein said plasticizer is selected from the group consisting of ethylene glycol, propylene glycol, 1,2-butylene glycol, 2,3-butylene glycol, styrene glycol, polyethylene glycol, glycol ether, propylene glycol monoethyl ether, ethylene glycol monoethyl ether, diethylene glycol monoethyl ether, sorbitol lactate, ethyl lactate, butyl lactate, ethyl glycolate, allyl glycolate, monoethanolamine, diethanolamine, triethanolamine, monisopropanolamine, triethylenetetramine, 2-amino-2-methyl-1,3-propanediol, triethyl citrate, triacetyl glycerol.

7. The transdermal patch for the use according to any one of claims 1 to 6, wherein said permeation enhancer is a monoterpene, a solvent, a lactam, a fatty acid, a fatty acid derivative, an amino acid derivative, α-tocopherol, and/or d-α-tocopheryl polyethylene glycol 1000 succinate.

8. The transdermal patch for the use according to claim 7, wherein said monoterpene is selected from the group consisting of
a. aliphatic monoterpenes, in particular myrcene, ocimene;
b. cyclic monoterpenes, in particular alpha-pinene, camphene, limonene, phellandrene, pinene, sabinene, terpinene;
c. aromatic monoterpenes, in particular cymene;
d. alcoholic monoterpenes, in particular borneol, carveol, dihydrocarveol, geraniol, menthol, linalool, perilla alcohol, sabinene hydrate, terpineol;
e. monoterpenes with carbonyl group, in particular camphor, menthone, carvone, citral, cuminaldehyde, dihydrocarvone, fenchone, safranal, thujone;
f. phenolic monoterpenes, in particular anethole, carvacrol, eugenol, eucalyptol, methyl cavicol, thymol, trans-anethole, picrocrocin;
g. monoterpenes-related compounds, in particular methylbutyryloxy-1-propenylbenzene, allyltetramethoxybenzene, anisaldehyde, anisketone, apiol, elemicine, hydroxyanetholmethylbutyric acid ester, zingerone, phenylpropanes, 5-Methoxyl-(2-Methylbutyryloxy)-1-propenylbenzene, allyltetramethoxybenzene, anethole, cuminaldehyde, elemicin, estragole, eugenol, eucalyptol, foeniculin, hydroxyanetholmethylbutyric acid ester, methylchavicol, myristicin, safrole, trans-Anethole, Zingeron.

9. The transdermal patch for the use according to claim 7, wherein said fatty acid or fatty acid derivative is oleic acid, dodecanol, sodium dodecyl sulphate, potassium dodecyl sulphtae, ammonium dodecyl sulphate, sodium octyl sulphate, potassium dodecyl sulphate, ammonium dodecyl sulphate isopropyl myristate, oleyl oleate, ethyl oleate, glycerol monolaurate, ascorbyl palmitate, sorbitan monolaurate, sorbitan monooleate, sorbitan monopalmitate, sorbitan trioleate, sorbitan monostearate, sorbitan tristearate, polyoxyethylene (20) sorbitan monolaurate, polyoxyethylene (20) sorbitan monopalmitate, polyoxyethylene (20) sorbitan monostearate.

10. The transdermal patch for the use according to claim 7, wherein said amino acid derivative is dodecyl 2-dimethylaminoproprionate, dodecyl N-acetylproline, N-laurylsarcosine, dodecyl 6-dimethylhexanoate.

11. The transdermal patch for the use according to any one of claims 1 to 10, wherein said matrix polymer is HPMC, and/or said plasticizer is PEG and/or said permeation enhancer is Geraniol.

12. The transdermal patch for the use according to any one of claims 1 to 11, wherein said hypomyelinating or demyelinating disease or condition is selected from the group consisting of leukodystrophies, demyelinating diseases of the central nervous system, multiple sclerosis, central pontine myelinolysis, glioma, schizophrenia, demyelination due to aging, diabetes or due to toxic agents, chronic inflammatory demyelinating polyneuropathy (CIDP), Charcot- Marie-Tooth disease (CMT), Guillain-Barre syndrome, hereditary neuropathy with liability to pressure palsy (HNPP), progressive inflammatory neuropathy, Dejerine- Sottas disease, Waardenburg syndrome, congenital hypomyelinating neuropathy (CHN), Cowchock syndrome, Rosenberg-Chutorian syndrome, or Roussy- Levy syndrome.

13. The transdermal patch for the use according to any one of claims 1 to 12, wherein said injury is selected from the group consisting of traumatic lesion of the nervous system, peripheral nerve injury or spinal cord injury.

14. A pharmaceutical composition, comprising aminophylline or theophylline, a matrix polymer, a plasticizer and a permeation enhancer for use as a medicament for promoting or accelerating myelination and/or remyelination use in the treatment or prevention of a hypomyelinating or a demyelinating disease or condition or a lesion of the peripheral or central nervous system where demyelination occurs.

15. The pharmaceutical composition for the use according to claim 14, wherein said matrix polymer is HPMC, and/or said plasticizer is PEG and/or said permeation enhancer is Geraniol.
